# EUROPEAN PATENT APPLICATION

(11) **EP 0 976 407 A1**
(43) Date of publication of application: **02.02.2000**
(21) Application number: 98919660.5
(22) Date of filing: 18.05.1998
(51) Int. Cl.: A61K 47/26

(54) **ANTISEPTIC COMPOSITION**

(30) Priority: 20.05.1997 JP 14731697; 15.05.1998 JP 13343898
(71) Applicant: Senju Pharmaceutical Co., Ltd., Osaka-shi, Osaka 541-0046 (JP)
(72) Inventor: NISHIHATA, Shuichi, Kobe-shi, Hyogo 651-2116 (JP); YAMAGUCHI, Masayo, Kobe-shi, Hyogo 651-1123 (JP); INADA, Katsuhiro, Kobe-shi, Hyogo 651-2242 (JP)
(74) Representative: Burford, Anthony Frederick
(86) International application number: JP9802210
(87) International publication number: WO9852612

(57) **Abstract**

A water-base antiseptic composition having a consistent and sufficient antiseptic effect and comprising an aqueous solution containing at least one component selected from the group consisting of hyaluronic acid, cromoglycic acid, flavin adenine dinucleotide and salts thereof and chlorhexidine gluconate, wherein the gluconate has such a concentration in the aqueous solution that it does not form any insoluble composite with the other components mentioned above and thus maintains the antiseptic effect.

## Description

The present invention relates to a composition made antiseptic by mixing chlorhexidine gluconate with a water-soluble liquid or an aqueous composition containing a pharmaceutical formulation that contains at least one component selected from hyaluronic acid, cromoglycic acid, flavine-adenine dinucleotide, and salts thereof, a method for producing the same, and a method of making said a water-soluble liquid or an aqueous composition antiseptic.

Hyaluronic acid, cromoglycic acid, flavine-adenine dinucleotide, and salts thereof are useful as pharmaceutical agents. For example, an aqueous eye drop formulation of hyaluronic acid and/or a salt thereof, especially sodium hyaluronate, is used, for example, in the field of ophthalmology for treatment and prevention of injuries on an eye tissue surface, for treatment of keratitis sicca, and as an intraocular infusion solution in inserting an intraocular lens in a cataract operation. In surgical areas, aqueous formulations of sodium hyaluronate are known to be effective for treatment of osteoarthritis of the knee and periarthritis of the shoulder, and these formulations are commercially available. Use of these is disclosed in US-A-4141973, US-A-4328803, JP-A- 60-84225, JP-A-60-31390, JP-A-61-28503, JP-A-01-800044, and JP-A-01-246227.

Sodium cromoglycate is also used as a pharmaceutical agent for treatment of allergic asthma or rhinitis. Flavine-adenine dinucleotide is used as eye drops for treatment of keratitis or blepharitis at the time of vitamin B₂ deficiency.

However, these pharmaceutical agents are liable to be contaminated and to degenerate in an aqueous formulation.

Chlorhexidine gluconate has been used as a preservative for aqueous formulations, especially as an antiseptic for aqueous formulations for local use. However, if it is added into an aqueous solution of the above-mentioned pharmaceutical agent, an insoluble complex is produced generating turbidity in the solution. Having regard to this incompatibility, chlorhexidine gluconate is not mixed with these pharmaceutical agents. . If it is mixed, the antiseptic effect of chlorhexidine gluconate is considered to be inhibited.

The present Inventors have found that a safe and useful aqueous formulation which contains at least one component selected from the group consisting of hyaluronic acid, cromoglycic acid, flavine-adenine dinucleotide, and salts thereof, and which has a stable and sufficient antiseptic effect while avoiding incompatibility.

The present Inventors have found that a stable and safe aqueous formulation having a sufficient antiseptic effect can be obtained by mixing chlorhexidine gluconate, which was usually considered as incompatible, in an amount that does not produce an insoluble complex.

The present invention provides a water-soluble antiseptic composition containing at least one component selected from hyaluronic acid, cromoglycic acid, flavine-adenine dinucleotide, and salts thereof and chlorhexidine gluconate, wherein the gluconate concentration is less than that which forms an insoluble complex with said component when the composition is added to water to form an aqueous solution; an aqueous antiseptic composition comprising an aqueous solution containing said component(s) and chlorhexidine gluconate, wherein the chlorhexidine gluconate in the aqueous solution has such a concentration that it does not form an insoluble complex with said component(s) while maintaining an antiseptic effect; and a process of making an aqueous composition containing said component(s) antiseptic by mixing with chlorhexidine gluconate in an amount less than that which forms an insoluble complex.

The concentration of the chlorhexidine gluconate in the composition of the present invention usually is 0.0001 to 0.05 wt%, preferably 0.001 to 0.01 wt%, more preferably around 0.005 wt%, if it is administered to a mucous membrane as eye drops, ear drops, or nose drops, though the concentration depends on the type and form of the main pharmaceutical agent and administration site.

The concentration of one or more components selected from hyaluronic acid, cromoglycic acid, flavine-adenine dinucleotide, and salts thereof in the composition of the present invention is determined in accordance with the intended use of the composition. The concentration is typically 0.001 to 5 wt%, preferably 0.005 to 3 wt%, more preferably 0.005 to 1 wt%.

The composition of the present invention can be administered in the form of various oral or non-oral liquid, preferably as a local external liquid, more preferably as eye drops, ear drops, or nose drops.

The composition of the present invention may contain another main pharmaceutical agent and/or a component that produces a different type of pharmaceutical effect as long as it does not spoil the purpose of the present invention.

Various additives that are usually used, such as a pH adjuster, a gelling agent, a stabilizer, a solubilizer, a surfactant, a sweetener, a buffering agent, a flavor, a suspending agent, an absorption promoter, a colorant, an isotonicity agent, an emulsifier, a thickening agent, a forming agent, a dispersing agent, a preservative, an aromatizing agent, a dissolving agent, a dissolving auxiliary, and a solvent may be used as appropriate into these formulations as long as the purpose of the present invention can be achieved.

The composition of the present invention maintains a sufficient antiseptic effect that satisfies the standard of microorganism test as defined in United States Pharmacopoeia (USP) 23, 〈51〉 ANTIMICROBIAL PRESERVATIVES-EFFECTIVENESS.

Examples of the antiseptic test are as follows.

| Prescription | Rp. 1 | Rp. 2 |
|---|---|---|
| Sodium hyaluronate | 0.15 g | 0.15 g |
| Sodium chloride | 0.75 | 0.75 |
| Potassium chloride | 0.16 | 0.16 |
| Disodium hydrogenphosphate | 0.08 | 0.08 |
| Sodium dihydrogenphosphate | In appropriate amount | In appropriate amount |
| Benzalkonium chloride | 0.005 | - |
| Chlorhexidine gluconate | - | 0.005 |
| Sterile purified water | In appropriate amount | In appropriate amount |
| | | Total Amount 100 ml (pH 7.0) |

Rp. 1 is a comparative using benzalkonium chloride as a preservative. Rp. 2 is a preparation of the present invention.

The above-mentioned two preparations were subjected to an antiseptic effect test according to the provisions of the United States Pharmacopoeia after being filtered with a 0.45 µm filter.

As to sodium hyaluronate, the one manufactured by Shiseido Co., Ltd. and having a molecular weight of 2,000,000 was used. The following results were obtained by the test that extended over four weeks.

| (Rp. 1) | | | | | |
|---|---|---|---|---|---|
| microorganism | | Number of formed colonies | | | |
| | Start time | After 1 week | After 2 weeks | After 3 weeks | After 4 weeks |
| S. aureus | 6 | n.d. | n.d. | n.d. | n.d. |
| E. coli. | 6 | n.d. | n.d. | n.d. | n.d. |
| P. aeruginosa | 6 | 4 | 5 | 4 | 4 |
| C. albicans | 5 | 1 | 1 | 1 | 1 |
| A. niger | 5 | 2 | 1 | 1 | 1 |
| Unit: logCFU/ml | | | | | |

| (Rp. 2) | | | | | |
|---|---|---|---|---|---|
| microorganism | | Number of formed colonies | | | |
| | Start time | After 1 week | After 2 weeks | After 3 weeks | After 4 weeks |
| S. aureus | 6 | 1 | n.d. | n.d. | n.d. |
| E. coli. | 6 | 2 | 2 | 2 | 2 |
| P. aeruginosa | 6 | 1 | 1 | 1 | n.d. |
| C. albicans | 5 | 2 | 2 | 2 | 1 |
| A. niger | 5 | 4 | 4 | 4 | 4 |
| Unit: logCFU/ml | | | | | |

As will be apparent from the above, the preparation using benzalkonium chloride (Rp. 1) does not meet the USP standard, and the preparation using chlorhexidine gluconate (Rp. 2) has been found to meet the USP standard.

### Examples

The present invention will now be illustrated in detail by way of examples, but these are not to be understood to limit the present invention.

### Example 1 Eye Drops

| | |
|---|---|
| Sodium hyaluronate | 0.1 g |
| Allantoin | 0.1 g |
| Boric acid | 1.8 g |
| Sodium Citrate | 0.2 g |
| Chlorhexidine gluconate | 0.0015 g |
| Borax | In appropriate amount |
| Sterile purified water | In appropriate amount |
| | Total amount 100 ml (pH 7.0) |

The above components are dissolved in an ordinary manner to prepare an aqueous formulation.

### Example 2 Eye Drops

| | |
|---|---|
| Sodium hyaluronate | 0.05 g |
| Aminoethylsulfonic acid | 0.5 g |
| Boric acid | 1.5g |
| Chlorhexidine gluconate | 0.0025 g |
| Borax | In appropriate amount |
| Sterile purified water | In appropriate amount |
| | Total amount 100 ml (pH 6.5) |

The above components are dissolved in an ordinary manner to prepare an aqueous formulation.

### Example 3 Eye Drops

| | |
|---|---|
| Sodium hyaluronate | 0.35 g |
| Boric acid | 1.25 g |
| Sodium chloride | 0.3 g |
| Chlorhexidine gluconate | 0.0025 g |
| Sodium hydroxide | In appropriate amount |
| Sterile purified water | In appropriate amount |
| | Total amount 100 ml (pH 6.0) |

The above components are dissolved in an ordinary manner to prepare an aqueous formulation.

### Example 4 Eye Drops

| | |
|---|---|
| Sodium hyaluronate | 0.005 g |
| Naphazoline hydrochloride | 0.003 g |
| Diphenhydramine hydrochloride | 0.1 g |
| Pyridoxine hydrochloride | 0.05 g |
| Boric acid | 1.8 g |
| Sodium edetate | 0.02 g |
| Chlorhexidine gluconate | 0.002 g |
| Sodium hydroxide | In appropriate amount |
| Hydrochloric acid | In appropriate amount |
| Sterile purified water | In appropriate amount |
| | Total amount 100 ml (pH 6.1) |

The above components are dissolved in an ordinary manner to prepare an aqueous formulation.

### Example 5 Nose drops

| | |
|---|---|
| Sodium hyaluronate | 0.01 g |
| Naphazoline hydrochloride | 0.05 g |
| Chlorpheniramine maleate | 0.05 g |
| Chlorhexidine gluconate | 0.005 g |
| Sodium citrate | 0.2 g |
| Concentrated glycerin | 2.1 g |
| Sodium hydroxide | In appropriate amount |
| Hydrochloric acid | In appropriate amount |
| Sterile purified water | In appropriate amount |
| | Total amount 100 ml (pH 6.0) |

The above components are dissolved in an ordinary manner to prepare nose drops.

### Example 6 Eye drops

| | |
|---|---|
| Sodium hyaluronate | 0.01 g |
| Flavine-adenine dinucleotide | 0.01 g |
| ε-aminocaproic acid | 0.1 g |
| Boric acid | 1.8 g |
| Chlorhexidine gluconate | 0.005 g |
| Sodium hydroxide | In appropriate amount |
| Sterile purified water | In appropriate amount |
| | Total amount 100 ml (pH 7.5) |

The above components are dissolved in an ordinary manner to prepare eye drops.

### Example 7 Nose drops

| | |
|---|---|
| Sodium hyaluronate | 0.01 g |
| Sodium cromoglycate | 1.0 g |
| Chlorhexidine gluconate | 0.002 g |
| Hydrochloric acid | In appropriate amount |
| Sterile purified water | In appropriate amount |
| | Total amount 100 ml (pH 5.0) |

The above components are dissolved in an ordinary manner to prepare nose drops

Each of the formulations obtained in the above-mentioned embodiments were a colorless transparent liquid at the time of preparation and even after being preserved at room temperature for three months.

According to the present invention, there is provided an antiseptic composition that overcomes the incompatibility problem and meets the United States Pharmacopoeia by mixing chlorhexidine gluconate with a water-soluble or aqueous composition containing hyaluronic acid, cromoglycic acid, flavine-adenine dinucleotide, or salts thereof.

## Claims

1. An aqueous antiseptic composition comprising an aqueous solution containing at least one component selected from hyaluronic acid, cromoglycic acid, flavine-adenine dinucleotide, and salts thereof and chlorhexidine gluconate, wherein the concentration of chlorhexidine gluconate in the aqueous solution is such that it does not form an insoluble complex with said component(s) while maintaining an antiseptic effect.

2. A composition as claimed in Claim 1, wherein at least one component is hyaluronic acid and/or a salt thereof.

3. A composition as claimed in Claim 1 or Claim 2, wherein the chlorhexidine gluconate concentration is 0.0001 to 0.05 wt%.

4. A composition as claimed in Claim 3, wherein the composition is selected from eye drops, ear drops, and nose drops and the chlorhexidine gluconate concentration is 0.001 to 0.01 wt%

5. A composition as claimed in Claim 4, wherein the chlorhexidine gluconate concentration is around 0.005 wt%.

6. A composition as claimed in any one of the preceding claims, wherein the concentration of said component(s) is 0.001 to 5 wt%.

7. A composition as claimed in Claim 6, wherein the concentration of said component(s) is 0.005 to 3 wt%.

8. A composition as claimed in Claim 7, wherein the concentration of said component(s) is 0.005 to 1 wt%.

9. A water-soluble antiseptic composition containing at least one component selected from hyaluronic acid, cromoglycic acid, flavine-adenine dinucleotide, and salts thereof and chlorhexidine gluconate, wherein the gluconate concentration is less than that which forms an insoluble complex with said component(s) when the composition is added to water to form an aqueous solution.

10. An antiseptic composition as claimed in Claim 9, wherein at least one component is hyaluronic acid and/or a salt thereof.

11. A process for producing an aqueous antiseptic composition by dissolving at least one component selected from hyaluronic acid, cromoglycic acid, flavine-adenine dinucleotide, and salts thereof in water and mixing with chlorhexidine gluconate at such a concentration that it does not form an insoluble complex with said component(s) in an aqueous solution while maintaining an antiseptic effect

12. A process as claimed in Claim 11, wherein the aqueous antiseptic composition is as defined in any one of Claims 2 to 8.

13. A process of making antiseptic an aqueous composition containing at least one component selected from hyaluronic acid, cromoglycic acid, flavine-adenine dinucleotide, and salts thereof, wherein the aqueous composition is mixed with chlorhexidine gluconate in an amount less than that which forms an insoluble complex.

14. A process as claimed in Claim 13, wherein at least one component is hyaluronic acid and/or a salt thereof.
